# EUROPEAN PATENT SPECIFICATION

(11) **EP 1 693 020 B1**
(45) Date of publication and mention of the grant of the patent: **06.06.2007**
(21) Application number: 06110144.0
(22) Date of filing: 20.02.2006
(51) Int. Cl.: A61B 19/02, B65D 77/04

(54) **Container for collecting and transporting special materials**
Behälter zum Sammeln und Transportieren von Sondermüll
Réceptacle collecteur et de transport de déchets spéciaux

(30) Priority: 21.02.2005 NL 1028352; 13.05.2005 NL 1029039; 08.09.2005 NL 1029903
(43) Date of publication of application: 23.08.2006
(73) Proprietor: Ecama Holding B.V., 3862 WL Nijkerk (NL)
(72) Inventor: Versluis, Evert, Cornelis, Antonie, 3862 WL, Nijkerk (NL)
(74) Representative: de Vries, Johannes Hendrik Fokke

(56) References cited:
- EP-A- 0 351 483
- EP-A- 1 472 987
- US-A- 5 160 025
- US-A- 5 427 238
- US-A- 5 687 839

## Description

The invention relates to a container for collecting and transporting special materials, such as, for example, hospital waste and diagnostic samples, according to the preamble of claim 1.

US-A-5 687 839 discloses a container of the above-mentioned type. In this known container, the primary holder includes a cylindrical body having a bottom and an open end adapted to receive a cap to be screwed on the open end. The liquid tightness and air tightness in this known container is to be provided by the secondary bag of fluid barrier located between the primary holder and the outer box.

US-A-5 160 025 discloses a battery shipping container for transporting wet batteries in aircraft. The container includes a closed, rigid outer box, wherein two sealable impermeable bags are provided for enclosing a battery to be transported.

US-A-5 427 238 discloses a mailer for sharp medical waste, comprising three separated parts. A first part comprises a primary container for receiving the sharps and is puncture resistant and leak-proof. A second part comprises a secondary containment system in which the primary container is received. The third part comprises an outer shipping container.

EP-A-0 351 483 discloses a container for hospital garbage formed by a rigid outer casing, a flexible envelop contained within the rigid outer casing, and an auxiliary rigid casing as primary holder located within the flexible envelop.

It has been found from experiments with the container shown in EP-A-1 472 987 that improvement is possible as regards the closure of the container and the liquid-tightness and airtightness. The known container does not, for example, meet the standards laid down in Packing Instruction P620.

The object of the invention is to provide an improved container of this type that can meet the strictest standards.

To this end, according to the invention, a container is provided, having the characterizing features of claim 1.

A container that meets very high standards as regards liquid-tightness and airtightness is obtained in this manner. It has been found that the container according to the invention is capable of withstanding a pressure difference of 95 kPa without any problem.

A container that fully meets Packing Instruction P620 is obtained in this manner.

According to a particularly advantageous embodiment, the outer box is designed with a rectangular open upper side and a cover which, in a closure position, can be placed on the outer box in order to close the outer box, at least one locking opening being provided in each of at least two opposite side walls, near the open upper side of the outer box, which locking opening is closed off on the inside by a box part that separates the secondary bag from the locking opening, and the cover having a peripheral edge which encloses the side walls of the outer box in the closure position of the cover and which bears a locking lip at the location of each locking opening, which locking lip can be pushed into the locking opening between the box part and the side wall of the outer box. In this embodiment the locking engagement of the cover is strengthened when there is an increase in the internal pressure of the container, owing to the fact that the secondary flexible bag wedges the locking lips between the box part and the side walls of the outer box.

The invention also provides a method for packing special materials, such as, for example, hospital waste and diagnostic samples, according to claim 13.

The invention will be explained in greater detail below with reference to the drawing, in which some embodiments of the container according to the invention are schematically shown.
Figure 1 is a perspective view of a first embodiment of the container according to the invention, in which the various parts of the container are shown in the disassembled state.
Figure 2 is a schematic cross-sectional view of the container of Figure 1, in the assembled state.
Figure 3 shows the primary holder of the container of Figure 1, with the primary flexible bag closed so as to be liquid-tight and airtight.
Figure 4 shows the intermediate box of the container of Figure 1, in the open state.
Figure 5 is a perspective view of the outer box with the corresponding cover of the container of Figure 1.
Figures 6A and 6B show schematically a locking lip and the placing of said locking lip in the locking opening.
Figure 7 shows the situation in which the locking lip is fully inserted into the locking opening.
Figure 8 shows an alternative embodiment of the container according to the invention.

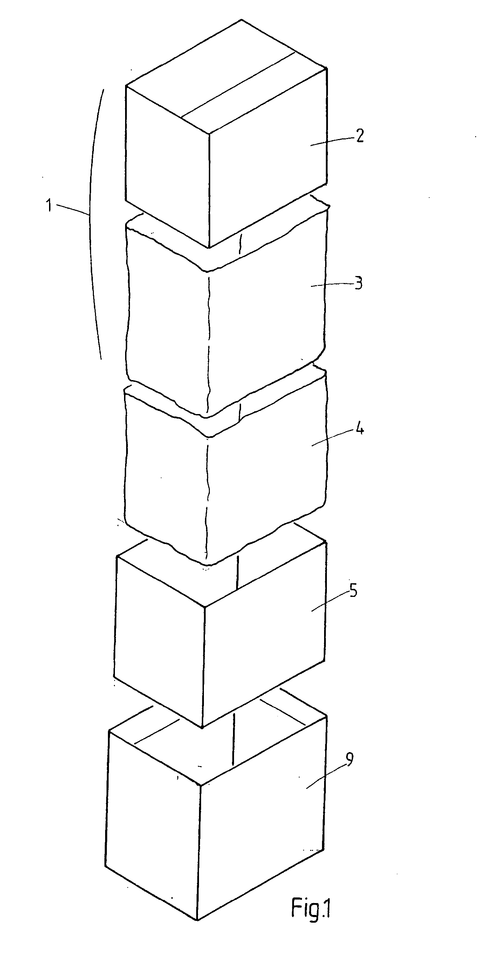

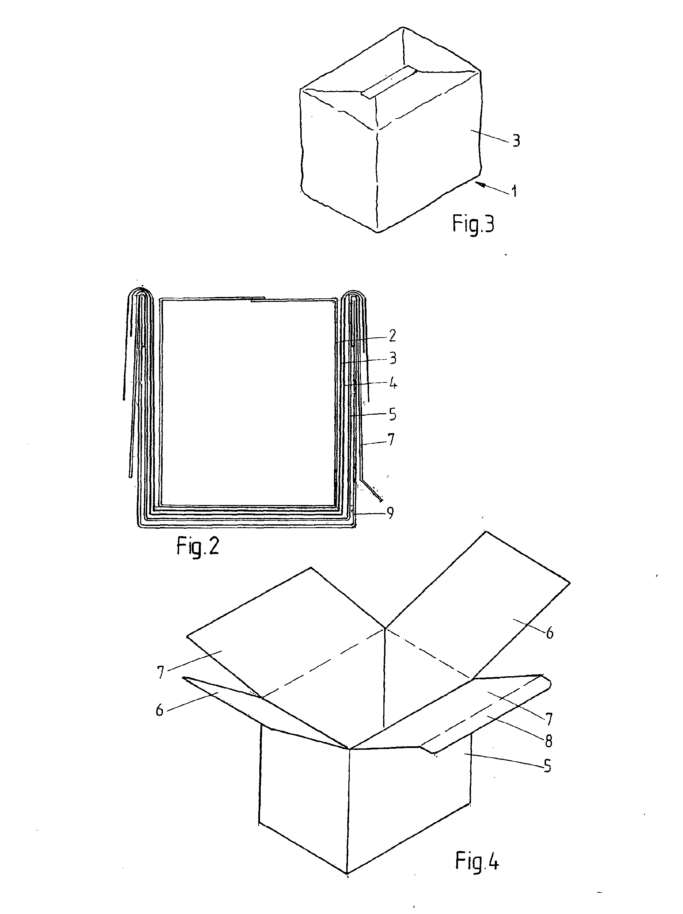

Figure 1 shows an embodiment of a container for collecting and transporting special materials, such as, for example, hospital waste and diagnostic samples, in the disassembled state. Figure 2 shows said container in cross section. The container shown is provided with a primary holder 1, which in the case of this embodiment consists of an inner box 2 and a primary flexible bag 3. Said primary flexible bag is made of a suitable material, such as, for example, latex, neoprene, chloroprene, PVC, HDPE, LDPE or the like, which is at least virtually airtight and liquid-tight, and which can also be closed in a suitable manner so as to be airtight and liquid-tight. This closure can be achieved, for example, by thermal welding or gluing, or by using suitable mechanical closure means. Figure 3 shows as an example the closed primary holder 1, with a schematically indicated mechanical closure means which provides an airtight and liquid-tight closure of the primary flexible bag 3.

The inner box 2 has an upper side and a bottom side with four flaps, the long flaps overlapping each other by several centimetres. A box with a closed top and bottom is obtained by folding the flaps in the plane of the upper and bottom sides. The material of the inner box 2 is of waterproof cardboard, for example SAPINO waterproof cardboard, 600 g/m². Of course, a different grade of cardboard or a different material can also be used, depending on the envisaged use of the container and the standards that have to be met by the container. The inner box 2 ensures that the primary holder 1 is puncture resistant, while it is ensured by means of the primary flexible bag 3 that the primary holder can be closed so as to be liquid-tight and airtight.

The primary holder 1, comprising the inner box 2 and the primary bag 3, is placed in a secondary holder 4, which in the embodiments described is in the form of a secondary flexible bag, which in the embodiments described meets the same specifications as the primary flexible bag. A suitable absorbent material (not shown) can be placed between the bags 3 and 4, which absorbent material absorbs escaping moisture in the event of leakage of the primary holder.

In the embodiment according to Figure 1 the assembly consisting of primary holder 1 and secondary holder 4 is placed in an intermediate box 5, which is shown in greater detail in Figure 4. The intermediate box 5 has a rectangular cross section and has top flaps 6, 7 and corresponding bottom flaps, which are not visible in Figure 4. Each flap 6, 7 has such dimensions that it fully covers the opening of the intermediate box 5, one of the flaps 7 also being provided with a closure flap 8, which during closure of the container falls between the opposite wall of an outer box 9 shown in Figure 1 and the outer side of the intermediate box 5. In the case of the embodiment described the intermediate box 5 is made of a waterproof cardboard, for example SAPINO waterproof cardboard, 1100 g/m². Of course, a different grade of cardboard or a different material can also be used, depending on the envisaged use of the container and the standards that have to be met by the container.

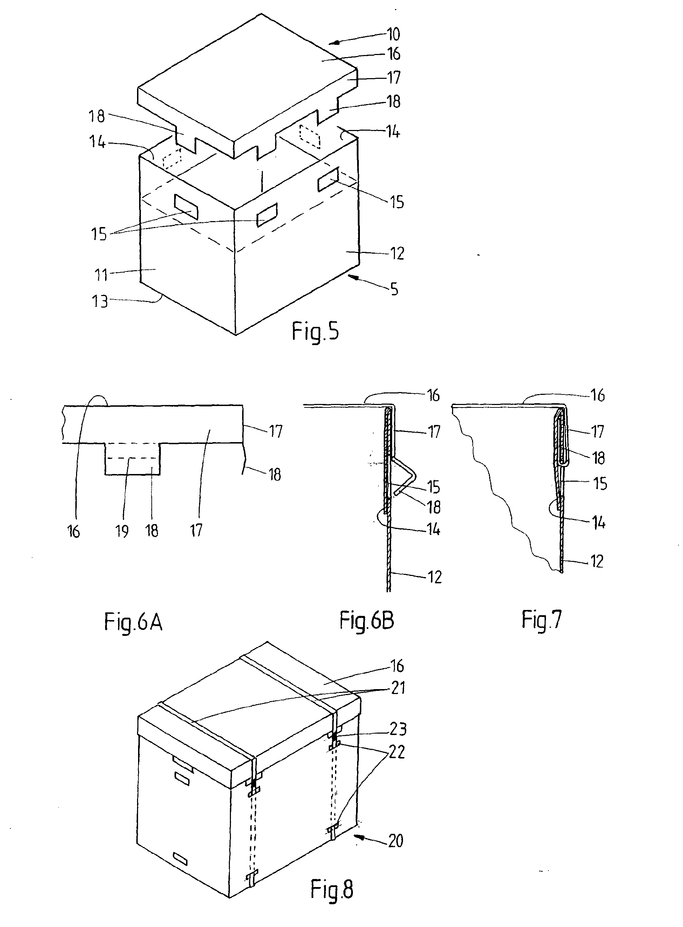

The intermediate box 5 with the primary holder 1 and secondary holder 4 placed therein are placed in the outer box 9, the intermediate box 5 being accommodated in the outer box 9 with a close fit. A schematic cross section of the assembled container is shown in Figure 2. The outer box 9 and a corresponding cover 10 are illustrated in greater detail in Figure 5, while Figures 6 and 7 show a number of details. The outer box 9 and the cover 10 in this case are made of the same material as the intermediate box 5 and, depending on the envisaged use, may be made of a heavier or a lighter grade of cardboard.

The outer box 9 has pairs of opposite side walls 11, 12, a closed bottom 13 and an open upper side. At the upper side the side walls have top flaps 14, which are folded inwards and lie against the inner side of the side walls 11, 12. A locking opening 15 is provided in each of the two short side walls 11, while two locking openings 15 are present in the two opposite long side walls 12. The locking openings 15 are covered by the uppers flaps 14 when the latter are folded inwards.

The cover 10 has a peripheral edge 17, which projects downwards from the upper surface 16 and which, in the closure position of the cover that is partially shown in Figure 7, encloses the side walls 11, 12. At the location of each locking opening 15, the peripheral edge 17 bears a locking lip 18, which can be pushed into the corresponding locking opening 15 between the downwardly folded top flap 14 and the side wall 11, 12 respectively of the outer box 9.

In the embodiment as shown, a fold line 19 is formed in each locking lip 18, in order to make it easier to push a locking lip 18 into the locking opening 15, as shown schematically in Figures 6A and 6B. If desired, two or more fold lines 19 may be disposed at regular intervals over the height of the locking lip 18.

In the container described with reference to Figures 1 - 7, the dimensions and/or the properties of the material of at least the secondary flexible bag are selected in such a manner that in the event of any excess pressure inside said bag 4, said bag 4 will lie with its entire external surface against the inner side of the intermediate box 5. Said intermediate box 5 in turn is placed in the outer box 9 with a close fit, so that in the event of increasing pressure the boxes 5, 9 will provide the mechanical strength to resist the excess pressure. The secondary flexible bag therefore provides the required airtight and liquid-tight properties, while the boxes 5, 9 deliver the required strength. In other words, in the event of excess pressure in the secondary flexible bag 4, said bag will interact with the intermediate box 5 and the outer box 9 to withstand the excess pressure. The primary bag 3 preferably has the same properties as the secondary bag 4, so that in the event of excess pressure in the primary bag, both bags will interact with the boxes 5 and 9, each of the two bags 3, 4 being capable of withstanding the excess pressure by interacting with the boxes 5, 9.

The above-described locking of the cover 10 on the outer box 9 with the aid of the locking lips 18 and locking openings 15 furthermore has the advantage that in the event of an increase in the internal pressure in the container the top flaps 14 will clamp the inserted locking lips 18 against the side walls 11, 12, with the result that the clamping force increases as the pressure increases.

In experiments carried out with the container as described herein it was found that an excess pressure of 95 kPa gives rise to no damage whatsoever. Moreover, the container described withstands the usual drop tests and bar tests that containers of this type must pass.

In an alternative embodiment the container may be produced without the intermediate box if the outer box 9 has sufficient strength for the purpose for which the container is intended. In the embodiment with the intermediate box 5 as described above, the top flaps 14 of the outer box 9 may be dispensed with if desired, since the vertical walls of the intermediate box 5 close off the locking openings 15. The locking lips 18 are pushed between the walls of the intermediate box 5 and the outer box 9 in this case, and with increasing pressure in the container are wedged between the walls of the intermediate box 5 and the outer box 9. In both variants it is therefore a box part, i.e. the vertical wall of the intermediate box 5 or the folded-over top flap 14 of the outer box 9, that separates the locking opening 15 from the secondary bag 4 and that clamps the locking lip 18 against the side wall 11, 12. This means that in both variants the outer box 9 is puncture resistant.

According to an advantageous embodiment of the invention, at least the secondary flexible bag 4, and preferably also the primary flexible bag 3, is so dimensioned that in the event of excess pressure of, for example, 95 kPa, if the intermediate box 5 and outer box 9 will give way, said bag or bags can expand, without becoming damaged, in order to lower the pressure inside the secondary flexible bag 4. As an alternative, the elasticity of the secondary and/or primary bag 4, 3 can also be such that said bag or bags can expand a number of times in order to lower the internal pressure, without giving way. It is further possible to achieve an expansion of the flexible bag by a combination of the dimensions and elasticity. By using a flexible bag 3, 4 which meets these standards it is ensured that if the outer box 9 and the intermediate box 5 unexpectedly give way, the bag 3, 4 can expand without giving way.

Figure 8 shows in perspective view an alternative embodiment of the container according to the invention, which is designed in substantially the same way as the container according to Figure 1. In this case an outer box 20 provided with closure tapes 21, shown schematically in Figure 8, is used. The closure tapes in this case extend over the locking lips 18 and locking openings 15 in the long side walls 12 of the outer box 9. If desired, the cover 10 can be designed without locking lips 18 and the outer box 9 can be designed without locking openings 15 respectively in this embodiment. As indicated by a dashed line in Figure 8, the closure tapes 21 are guided partially along the inner side of the long side walls 12, for which purpose grooves 22 have been provided in said side walls 12. Each closure tape 21 has a closure element such as a clasp or the like, which is indicated schematically by 23 in Figure 8.

In the embodiments described the cover 10 is fixed on the outer box 9, 20 by the locking lips 18 alone or by the closure tapes as well. As an alternative, it is also possible to provide an adhesive strip (not shown) on the inner side of the cover 10 along the peripheral edge 17, against which adhesive strip the top edge of the outer box 9, 20 can be pressed.

It will be apparent from the foregoing that the invention provides a pressure-resistant container, which can withstand high internal pressures of 95 kPa or more. Furthermore, the container described has a liquid-tight and airtight primary holder 1 and a liquid-tight and airtight secondary holder 4. The primary holder 4 is also puncture resistant. The selected dimensions and/or the material properties enable at least said secondary holder 4, and preferably also the primary bag 3, to interact with the outer box 9, possibly with the interposition of an intermediate box 5, in such a manner that the container can withstand a high internal pressure.

The invention is not restricted to the embodiments described above, which can be varied in several ways without departing from the scope of the claims.

## Claims

1. A container for collecting and transporting special materials, such as, for example, hospital waste and diagnostic samples, which container comprises a primary holder (1), a secondary holder (4) and a closed, rigid outer box (9), wherein the secondary holder comprises a secondary flexible bag (4) which can be closed in such a manner that it is at least substantially liquid-tight and airtight, and the dimensions of which, at least in the event of internal excess pressure, are at least equal to the internal dimensions of the rigid outer box, wherein in the event of excess pressure in the secondary flexible bag said flexible bag and the closed outer box interact in order to withstand the excess pressure, **characterized in that** the primary holder (1) comprises an inner box (2) and a primary flexible bag (3) enclosing the inner box, which inner box has a closed bottom and upper wall, the primary flexible bag being closable so that it is at least substantially liquid-tight and airtight, which primary holder is accommodated in the secondary flexible bag and is accommodated together with the secondary bag in the outer box with a close fit.

2. A container according to claim 1, wherein the primary holder accommodated in the secondary flexible bag is placed in an intermediate box (5), which is accommodated in the outer box (9) with a close fit.

3. A container according to claim 2, wherein the intermediate box (5) is provided with top and bottom flaps (6, 7), which each fully cover the rectangular opening surface of the intermediate box, wherein a top flap (7) preferably has a closure lip (8) which can be inserted between the outer side of the intermediate box and the inner side of the outer box (9).

4. A container according to one of the preceding claims, wherein the outer box (9) is designed with a rectangular open upper side and a cover (10) which, in a closure position, can be placed on the outer box in order to close the outer box, at least one locking opening (15) being provided in each of at least two opposite side walls (11, 12), near the open upper side of the outer box, which locking opening is closed off on the inside by a box part (14) that separates the secondary bag (4) from the locking opening, and the cover (10) having a peripheral edge (17) which, in the closure position of the cover, encloses the side walls (11, 12) of the outer box (9) and which, at the location of each locking opening, bears a locking lip (18), which locking lip can be pushed into the locking opening between the box part and the side wall of the outer box.

5. A container according to claim 4, wherein an adhesive strip is provided on the inner side of the cover (10) near the peripheral edge (17), wherein the top edge of the outer box (9) projects into the adhesive strip if the cover is pressed into the closure position on the outer box.

6. A container according to claim 4 or 5, wherein each locking lip (18) is provided with one or more fold lines (19).

7. A container according to any one of the preceding claims, wherein the dimensions and/or the elasticity of the primary and/or secondary flexible bag (3, 4) are selected in such a manner that in the absence of the outer box (9) an expansion of the primary and/or secondary bag is possible in order to release excess pressure in the primary and/or secondary bag.

8. A container according to claim 7, wherein the dimensions and/or elasticity of the primary and/or secondary flexible bag (3, 4) permit an expansion of at least five times their size.

9. A container according to any one of the preceding claims, wherein one or more closure tapes (21) are provided, which closure tapes enclose the outer box (9) and the cover (10) placed in the closure position, each closure tape preferably running over locking openings (15) provided in opposite side walls (12).

10. A container according to claim 9, wherein the closure tapes (21) run over part of the height of the side walls (12) of the outer box (9) on the inner side thereof.

11. A container according to any one of the claims 2 - 10, wherein the inner box (2) of the primary holder (1) is provided with flattened box corner points.

12. A container according to one of the preceding claims, wherein a liquid-absorbing material is inserted between the primary and secondary holders (1, 4).

13. A method for packing special materials, such as, for example, hospital waste and diagnostic samples, wherein the special material is placed in the liquid-tight primary holder (1) of a container according to one of the preceding claims, **characterized in that** the flexible secondary bag (4) is closed so as to be at least substantially liquid-tight and airtight and the rigid outer box (9) is then closed by pressing the cover (10) onto the outer box in the closure position, after which each locking lip (18) of the cover is pushed into the corresponding locking opening (15) between the box part and the side wall of the outer box, wherein the primary holder (1) comprises an inner box (2) and a flexible bag (3) enclosing the inner box, wherein prior to the closure of the secondary flexible bag the primary flexible bag is closed in such a manner that it is at least substantially liquid-tight and airtight.

14. A method according to claim 13, wherein the primary holder (1) accommodated in the secondary flexible bag (4) is placed in an intermediate box (5), and wherein after closure of the secondary bag the top flaps (6, 7) of the intermediate box are closed and the closure lip (8) is inserted between the outer side of the intermediate box and the inner side of the outer box (9).

## Patentansprüche

1. Behälter zum Sammeln und Transportieren von Sondermüll, wie beispielsweise Krankenhausmüll und Diagnoseproben, wobei der Behälter ein Primärbehältnis (1), ein Sekundärbehältnis (4) und eine geschlossene steife Außenschachtel (9) aufweist, wobei das Sekundärbehältnis einen flexiblen Sekundärbeutel (4) aufweist, der derart verschlossen werden kann, dass er zumindest im Wesentlichen wasserdicht und luftdicht ist, und von dem die Abmessungen zumindest im Fall von Innenüberdruck wenigstens gleich den Innenabmessungen der steifen Außenschachtel sind, wobei im Fall von Überdruck in dem flexiblen Sekundärbeutel der flexible Beutel und die geschlossene Außenschachtel zusammenwirken, um dem Überdruck standzuhalten,
**dadurch gekennzeichnet, dass**
das Primärbehältnis (1) eine Innenschachtel (2) und einen flexiblen Primärbeutel (3) aufweist, der die Innenschachtel umgibt, wobei die Innenschachtel eine geschlossene untere und obere Wandung aufweist und der flexible Primärbeutel so verschließbar ist, dass er zumindest im Wesentlichen wasserdicht und luftdicht ist, wobei das Primärbehältnis in dem flexiblen Sekundärbeutel untergebracht ist und zusammen mit dem Sekundärbeutel eng anliegend in der Außenschachtel untergebracht ist.

2. Behälter nach Anspruch 1, wobei das in dem flexiblen Sekundärbeutel untergebrachte Primärbehältnis in eine Zwischenschachtel (5) eingebracht ist, die in der Außenschachtel (9) eng anliegend untergebracht ist.

3. Behälter nach Anspruch 2, wobei die Zwischenschachtel (5) mit oberen und unteren Klappen (6, 7) versehen ist, die jeweils die rechteckige Öffnungsfläche der Zwischenschachtel vollständig überdecken, wobei vorzugsweise eine obere Klappe (7) eine Verschlusslippe (8) aufweist, die zwischen die Außenseite der Zwischenschachtel und die Innenseite der Außenschachtel (9) eingeschoben werden kann.

4. Behälter nach einem der vorhergehenden Ansprüche, wobei die Außenschachtel (9) mit einer rechteckigen offenen Oberseite und einem Deckel (10) ausgebildet ist, der in einer Schließposition auf der Außenschachtel platziert werden kann, um die Außenschachtel zu verschließen, wobei zumindest eine Verriegelungsöffnung (15) in jeder von zumindest zwei gegenüberliegenden Seitenwänden (11, 12) in der Nähe der offenen Oberseite der Außenschachtel vorgesehen ist, wobei die Verriegelungsöffnung auf der Innenseite durch einen den Sekundärbeutel (4) von der Verriegelungsöffnung separierenden Schachtelabschnitt (14) versperrt wird, und die Abdeckung (10) einen Umfangsrand (17) aufweist, der in der Schließposition des Deckels die Seitenwände (11, 12) der Außenschachtel (9) umgibt und der an den Positionen jeder Verriegelungsöffnung einen Verriegelungsansatz (18) aufweist, wobei der Verriegelungsansatz in die Verriegelungsöffnung zwischen dem Schachtelabschnitt und der Seitenwandung der Außenschachtel gedrückt werden kann.

5. Behälter nach Anspruch 4, wobei ein Klebestreifen auf der Innenseite des Deckels (10) in der Nähe des Umfangsrandes (17) vorgesehen ist, wobei der obere Rand der Außenschachtel (9) bis zu dem Klebestreifen hervorsteht, wenn der Deckel auf die Außenschachtel in die Schließposition gedrückt wird.

6. Behälter nach Anspruch 4 oder 5, wobei jeder Verriegelungsansatz (18) mit einer oder mehreren Falzlinien (19) versehen ist.

7. Behälter nach einem der vorhergehenden Ansprüche, wobei die Abmessungen und/oder die Elastizität des flexiblen Primär- und/oder flexiblen Sekundärbeutels (3, 4) derart ausgewählt sind, dass beim Nichtvorhandensein der Außenschachtel (9) eine Ausdehnung des Primär- und/oder Sekundärbeutels möglich ist, um Überdruck in dem Primär- und/oder Sekundärbeutel abzubauen.

8. Behälter nach Anspruch 7, wobei die Abmessungen und/oder die Elastizität des flexiblen Primär- und/oder flexiblen Sekundärbeutels (3, 4) eine Ausdehnung von wenigstens dem fünffachen ihrer Größe zulassen.

9. Behälter nach einem der vorhergehenden Ansprüche, wobei ein oder mehrere Verschlussbänder (21) vorgesehen sind, wobei die Verschlussbänder die Außenschachtel (9) und den in der Schließposition angeordneten Deckel (10) umgeben, wobei jedes Verschlussband vorzugsweise durch Verriegelungsöffnungen (15) verläuft, die in gegenüberliegenden Seitenwänden (12) vorgesehen sind.

10. Behälter nach Anspruch 9, wobei die Verschlussbänder (21) längs der Höhe der Seitenwände (12) der Außenschachtel (9) auf deren Innenseite verlaufen.

11. Behälter nach einem der Ansprüche 2 bis 10, wobei die Innenschachtel (2) des Primärbehältnisses mit glatten Schachteleckpunkten versehen ist.

12. Behälter nach einem der vorhergehenden Ansprüche, wobei ein flüssigkeitsabsorbierendes Material zwischen das Primär- und das Sekundärbehältnis (1, 4) eingefügt ist.

13. Verfahren zum Verpacken von Sondermüll, wie beispielsweise Krankenhausmüll und Diagnoseproben, wobei der Sondermüll in dem wasserdichten Primärbehältnis (1) eines Behälters gemäß einem der vorhergehenden Ansprüche angeordnet wird, **dadurch gekennzeichnet, dass** der flexible Sekundärbeutel (4) so verschlossen wird, dass er zumindest im Wesentlichen wasserdicht und luftdicht ist, und dann die steife Außenschachtel (9) durch Andrücken des Deckels (10) auf die Außenschachtel in die Schließposition verschlossen wird, nachdem jeder Verriegelungsansatz (18) des Deckels in die entsprechende Verriegelungsöffnung (15) zwischen dem Schachtelabschnitt und der Seitenwandung der Außenschachtel gedrückt wird, wobei das Primärbehältnis (1) eine Innenschachtel (2) und einen flexiblen Beutel (3) aufweist, der die Innenschachtel umgibt, wobei der flexible Primärbeutel vor dem Verschließen des flexiblen Sekundärbeutels derart verschlossen wird, dass er zumindest im Wesentlichen wasserdicht und luftdicht ist.

14. Verfahren nach Anspruch 13, wobei das in dem flexiblen Sekundärbeutel (4) untergebracht Primärbehältnis (1) in der Zwischenschachtel (5) angeordnet wird, und wobei die oberen Klappen (6, 7) der Zwischenschachtel nach dem Verschließen des Sekundärbeutels geschlossen werden und die Verschlusslippe (8) zwischen die Außenseite der Zwischenschachtel und die Innenseite der Außenschachtel (9) eingebracht wird.

## Revendications

1. Récipient pour la collecte et le transport de matériaux spéciaux, tels que, par exemple, des déchets hospitaliers et des échantillons pour diagnostic, lequel récipient comprend un premier support (1), un second support (4) et une boîte externe rigide fermée (9), dans lequel le second support comprend un second sac flexible (4) qui peut être fermé de manière à être au moins sensiblement étanche aux liquides et étanche à l'air, et dont les dimensions, au moins dans le cas d'un excès de pression interne, sont au moins égales aux dimensions internes de la boîte externe rigide, dans lequel dans le cas d'un excès de pression dans le second sac flexible ledit sac flexible et la boîte externe fermée interagissent de manière à résister à l'excès de pression, **caractérisé en ce que** le premier support (1) comprend une boîte interne (2) et un premier sac flexible (3) entourant la boîte interne, laquelle boîte interne présente des parois supérieure et inférieure fermées, le premier sac flexible pouvant être fermé de manière à être au moins sensiblement étanche aux liquides et étanche à l'air, lequel premier conteneur est logé dans le second sac flexible et est logé avec le second sac dans la boîte externe avec un ajustement serré.

2. Récipient selon la revendication 1, dans lequel le premier support logé dans le second sac flexible est placé dans une boîte intermédiaire (5), qui est logée dans la boîte externe (9) avec un ajustement serré.

3. Récipient selon la revendication 2, dans lequel la boîte intermédiaire (5) est pourvue de rabats inférieurs et supérieurs (6, 7), qui recouvrent chacun entièrement la surface d'ouverture rectangulaire de la boîte intermédiaire, dans lequel un rabat supérieur (7) présente de préférence une lèvre de fermeture (8) qui peut être insérée entre le côté externe de la boîte intermédiaire et le côté interne de la boîte externe (9).

4. Récipient selon l'une des revendications précédentes, dans lequel la boîte externe (9) est conçue avec un côté supérieur ouvert et un couvercle (10) qui, dans une position de fermeture, peut être placé sur la boîte externe afin de fermer ladite boîte externe, au moins une ouverture de verrouillage (15) étant prévue dans chacune d'au moins deux parois latérales opposées (11, 12), à proximité du côté supérieur ouvert de la boîte externe, laquelle ouverture de verrouillage est fermée à l'intérieur par une partie de boîte (14) qui sépare le second sac (4) de l'ouverture de verrouillage, et le couvercle (10) présentant un bord périphérique (17) qui, dans la position de fermeture du couvercle, entoure les parois latérales (11, 12) de la boîte externe (9) et qui, au niveau de l'emplacement de chaque ouverture de verrouillage, comporte une lèvre de verrouillage (18), laquelle lèvre de blocage peut être poussée dans l'ouverture de verrouillage entre la partie de boîte et la paroi latérale de la boîte externe.

5. Récipient selon la revendication 4, dans lequel une bande adhésive est prévue sur le côté interne du couvercle (10) à proximité du bord périphérique (17), dans lequel le bord supérieur de la boîte externe (9) fait saillie sur la bande adhésive si le couvercle est pressé en position de fermeture sur la boîte externe.

6. Récipient selon la revendication 4 ou 5, dans lequel chaque lèvre de verrouillage (18) est pourvue d'une ou de plusieurs lignes de pliage (19).

7. Récipient selon l'une quelconque des revendications précédentes, dans lequel les dimensions et/ou l'élasticité des premier et/ou second sacs flexibles (3, 4) sont choisies d'une telle manière qu'en l'absence de la boîte externe (9) une expansion des premier et/ou second sacs flexibles est possible afin de libérer l'excès de pression dans le premier et/ou le second sacs flexibles.

8. Récipient selon la revendication 7, dans lequel les dimensions et/ou l'élasticité des premier et/ou second sacs flexibles (3, 4) permettent une expansion d'au moins cinq fois leur taille.

9. Récipient selon l'une quelconque des revendications précédentes, dans lequel un ou plusieurs rubans de fermeture (21) sont prévus, lesquels rubans de fermeture entourent la boîte externe (9) et le couvercle (10) placés dans la position de fermeture, chaque ruban de fermeture débordant de préférence sur des ouvertures de verrouillage (15) prévues dans des parois latérales opposées (12).

10. Récipient selon la revendication 9, dans lequel les rubans de fermeture (21) débordent sur une partie de la hauteur des parois latérales (12) de la boîte externe (9), sur le côté interne de celle-ci.

11. Récipient selon l'une quelconque des revendications 2 à 10, dans lequel la boîte interne (2) du premier support (1) est pourvue de points d'angle de boîte aplatis.

12. Récipient selon l'une des revendications précédentes, dans lequel un matériau absorbeur de liquide est inséré entre les premier et second supports (1, 4).

13. Procédé d'emballage de matériaux spéciaux, tels que, par exemple, des déchets hospitaliers et des échantillons pour diagnostic, dans lequel le matériau spécial est placé dans le premier support (1) étanche aux liquides d'un récipient selon l'une des revendications précédentes, **caractérisé en ce que** le second sac flexible (4) est fermé de manière à être au moins sensiblement étanche aux liquides et étanche à l'air et la boîte externe rigide (9) est ensuite fermée en pressant le couvercle (10) sur la boîte externe dans la position de fermeture, après quoi chaque lèvre de verrouillage (18) du couvercle est poussée dans l'ouverture de verrouillage correspondante (15) entre la partie de boîte et la paroi latérale de la boîte externe, dans lequel le premier support (1) comprend une boîte interne (2) et un sac flexible (3) entourant la boîte interne, le premier sac flexible étant fermé avant la fermeture du second sac flexible de manière à être au moins sensiblement étanche aux liquides et étanche à l'air.

14. Procédé selon la revendication 13, dans lequel le premier support (1) logé dans le second sac flexible (4) est placé dans une boîte intermédiaire (5), et dans lequel après la fermeture du second sac les rabats supérieurs (6, 7) de la boîte intermédiaire sont fermés et la lèvre de fermeture (8) est insérée entre le côté externe de la boîte intermédiaire et le côté interne de la boîte externe (9).
